# EUROPEAN PATENT APPLICATION

(11) **EP 4 648 062 A1**
(43) Date of publication of application: **12.11.2025**
(21) Application number: 24174802.9
(22) Date of filing: 08.05.2024
(51) Int. Cl.: G16H 50/20, G16H 40/67, G16H 50/80

(54) **IDENTIFICATION OF DISEASE ONSET BY MODELING SUBTLE PHYSIOLOGICAL CHANGES USING WEARABLE DEVICES**

(71) Applicant: Koninklijke Philips N.V., 5656 AG Eindhoven (NL)
(72) Inventor: LIU, Luoluo, Eindhoven (NL); SALVATI, Emmanuele, Eindhoven (NL); CONROY, Bryan, 5656AG Eindhoven (NL); MCFARLANE, Daniel Craig, Eindhoven (NL)
(74) Representative: Philips Intellectual Property & Standards

(57) **Abstract**

A system (1) includes an electronic processor (16, 20) integrated with or in wireless communication with an associated monitoring device (10). The electronic processor is programmed to receive physiological sensor data (13) of an associated human subject measured using the associated monitoring device (10); determine whether the associated human subject has indicia of onset an illness by applying a pre-trained artificial intelligence (Al) model (24) to the received physiological sensor data; and output a warning (26) in response to a determination that the associated human subject has indicia of onset of the illness. The pre-trained Al model is pre-trained to determine whether the human subject has received a vaccination.

## Description

This invention was made with government support under contract number HDTRA121C0006 awarded by the Defense Threat Reduction Agency, which is part of US Department of Defense. The government has certain rights in the invention.

### FIELD

The following relates generally to the medical monitoring arts, wearable medical monitor arts, early disease onset detection arts, and related arts.

### BACKGROUND

Health-related unobtrusive sensing systems enable replacement of continued hospitalization with unobtrusive vital signs sensor technologies, centered around the individual, to provide remote monitoring of the subject's general health condition. Vital signs monitoring typically includes monitoring one or more of the following physical parameters: heart rate (HR), blood pressure (BP), respiratory rate (RR), core body temperature and blood oxygenation (SpOz).

Physiological unobtrusive sensing systems are typically used to provide early detection of worsening of chronic medical conditions. Unobtrusive vital sign monitoring can additionally or alternatively detect an onset of a new health condition. A person can wear such a sensing system to detect onset of a contagious (i.e., communicable) disease (e.g., COVID-19), thus enabling timely quarantine. In a military context where unit readiness can be critical, early detection of onset of a communicable disease such as COVID can facilitate rapid isolation of infected personnel and limit spread of the disease throughout the unit. The effectiveness of this approach is greatly enhanced by detecting the disease in its onset phase before the individual becomes highly contagious.

The following discloses certain improvements.

### SUMMARY

In one aspect, a system includes an electronic processor integrated with or in wireless communication with an associated monitoring device. The electronic processor is programmed to receive physiological sensor data of an associated human subject measured using the associated monitoring device; determine whether the associated human subject has indicia of onset an illness by applying a pre-trained artificial intelligence (AI) model to the received physiological sensor data; and output a warning in response to a determination that the associated human subject has indicia of onset of the illness. The pre-trained AI model is pre-trained to determine whether the human subject has received a vaccination.

In another aspect, a vaccination status monitoring method includes receiving physiological sensor data of an associated human subject measured using an associated monitoring device; determining whether the associated human subject has indicia of onset an illness by applying a pre-trained AI model to the received physiological sensor data; and outputting a warning in response to a determination that the associated human subject has indicia of onset of the illness. The pre-trained AI model is pre-trained to determine whether the human subject has received a vaccination.

One advantage resides in providing a process to detect the subtle physiological changes from the effects of different vaccination groups including a placebo group.

Another advantage resides in excluding self-reported infections by patients in a vaccination study.

Another advantage resides in determining a type of vaccination given to a patient in a patient vaccination study.

Another advantage resides in generating a representation of true positive vaccination rates in a vaccination study.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIG. 1 diagrammatically shows a monitoring system in accordance with the present disclosure.
FIGS 2 and 3 shows example flow charts of operations suitably performed by the system of FIG. 1.

### DETAILED DESCRIPTION

As used herein, the singular form of "a," "an," and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

A challenge with using unobtrusive physiological monitoring systems for early detection of onset of a communicable disease is that the vital sign changes may be subtle and difficult to detect. One way to deal with this might be to train an artificial intelligence (AI) model to detect these subtle changes. However, it may be difficult to obtain a sufficient quantity of suitable training data for training such an AI model. A portion or all of the relevant training data would typically need to be acquired before the training subject becomes symptomatic. Hence, to collect the training data a large cohort of healthy individuals would need to be monitored and physiological data collected continuously, along with obtaining information on when specific individuals become symptomatic (of which this would be a subset of the total cohort as only a subset would be expected to contract the communicable disease). This would then be followed by retrospective analysis of the data to select training data from the relevant time period preceding the symptomatic phase. The approach would also rely upon accurate diagnosis of the communicable disease of interest in the cohort of training subjects. The foregoing process presents numerous practical difficulties and limitations.

In embodiments disclosed herein, physiological reaction to a vaccination is leveraged to provide suitable training data in a simpler and more controllable fashion. Some studies have been developed to determine whether patients have been given a vaccination (e.g., Pneumococcal vaccines, typhoid vaccines, COVID-19 vaccines, and so forth) or a saline water placebo. Since a presence of a vaccination in a patient triggers an immune response in the patient without severe symptoms onsets, it makes vaccination studies a good candidate for early prediction of various infection types. Side effects of pneumococcal vaccinations reported by the Centers for Disease Control and Prevention (CDC) are redness or pain where the shot is given, feeling tired, fever, or muscle aches (see, e.g., Pneumococcal Polysaccharide Vaccine Information Statements, Centers for Disease control and Prevention, https://www.cdc.gov/vaccines/hcp/vis/vis-statements/ppv.html). Side effects of the typhoid inactive vaccine from the CDC include pain from the shot, redness, or swelling at the site of the injection, fever, headache, and general discomfort (see e.g., Typhoid Vaccine Information Statements, Centers for Disease control and Prevention, https://www.cdc.gov/vaccines/hcp/vis/vis-statements/typhoid.html).

Thus, in embodiments disclosed herein, training data for training an AI model for early detection of a communicable disease of interest are collected from individuals who receive a vaccination for the communicable disease of interest (or, in some embodiments, a vaccination for another communicable disease with similar early onset symptoms). The vaccination serves as a surrogate for contraction of the actual disease, and the AI component is trained to detect the vaccination. The resulting trained AI model is then expected to also be useful to detect early onset of the disease in a monitored subject, under the expectation that the early onset disease will manifest in the subject's vital signs similarly to the vaccination. Advantageously, the time of the vaccination (e.g., an injection) is precisely known, as is the composition of the vaccine (thus, the "diagnosis" of the disease is known *a priori*)*.* In this approach, a cohort of subjects is created in a controlled fashion. Each subject of the cohort receives the vaccination at a known time, and wears the unobtrusive vital sign monitoring device for a suitable time period bracketing the time of vaccination. Thus, the cohort can have a smaller number of training subjects since every training subject receives the vaccination, and the training subjects can wear the unobtrusive vital sign monitoring device for a shorter (and well-defined) time interval.

One potential problem with this approach for collecting training data is that each training subject is aware of precisely when he or she receives the vaccination, and therefore could have certain psychosomatic symptoms in response to the vaccination. These psychosomatic symptoms present as real physiological changes and hence are measured by the wearable physiological monitoring device. However, the psychosomatic symptoms are a product of the person's psychological state, rather than being due to a reaction to the active agent of the vaccine (e.g., a dose of weakened or killed microorganism such as a virus or bacterium that in its live state may cause the communicable disease). For example, if the training subject is warned that they may experience aoura fever in response to the vaccination, they may actually have a fever as a psychosomatic symptom, even if the active agent in the vaccination would not have actually produced a fever in this particular training subject. Since in the case of disease onset (as opposed to vaccination) the person is initially unaware of the disease onset, psychosomatic symptoms are not expected to be present in disease onset. Consequently, psychosomatic symptoms in a training subject in response to a vaccination constitute a noise or error signal in the vaccination-based training data.

In some embodiments, a control training cohort is provided in a double-blind fashion to account for psychosomatic symptoms. In these embodiments, a certain fraction of the training subjects receive a placebo rather than a true vaccination. For example, if the vaccination is delivered as an intravenous injection, then the placebo could be a saline injection without the active agent of the vaccine. If this is done in a double-blind fashion, then neither the training subject nor the medical professional performing the vaccination knows whether it is a true vaccination containing the active agent, or a placebo injection. The training data is labeled by an anonymized identifier such as a dose serial number, and only after the training data are collected is the double-blind lifted to enable labeling of the training data as vaccination or placebo. The AI model is then trained to detect the true vaccination while not detecting the placebo. To ensure the training cohort is ultimately fully vaccinated, after the double-blind is lifted those training subjects who received the placebo can receive follow-up true vaccination. (For ethical reasons, the training subjects would typically be notified that there is a possibility that they may initially be receiving a placebo, but since no training subject knows specifically whether he or she is receiving a vaccination or placebo this general knowledge does not impact the results).

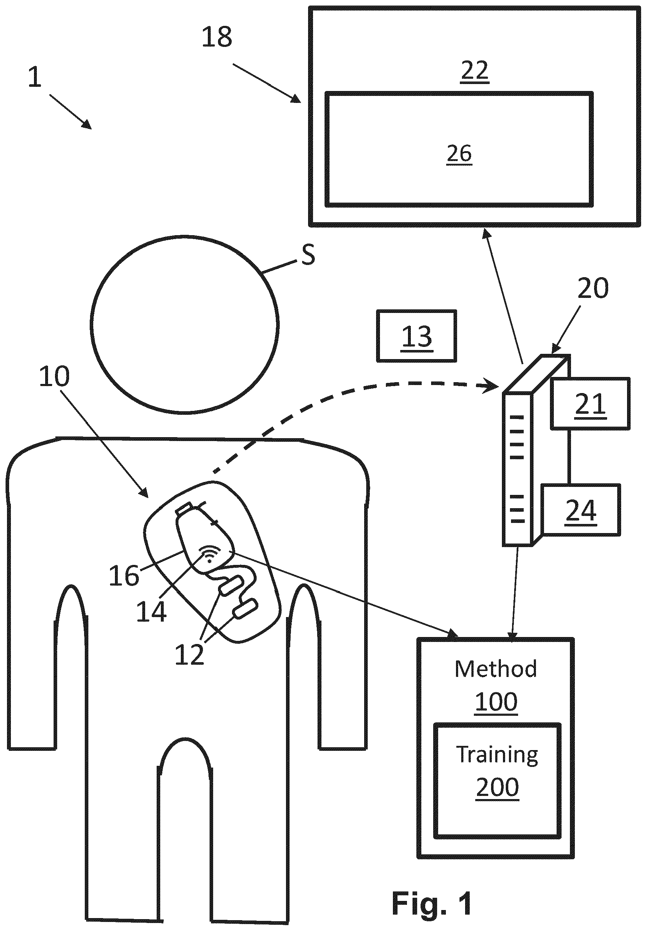

With reference to FIG. 1, a monitoring system 1 for monitoring an associated human subject S is shown. During the inference phase (using the trained AI model to detect early onset of a communicable disease of interest), the term "human subject" (and variants thereof) suitably refers to, and includes, an outpatient, a discharged patient, a patient undergoing screening using the monitoring device as part of an annual medical checkup, a healthy person wearing a vital sign monitoring device such as a vital sign measurement-equipped smart watch for precautionary purposes, a member of a military unit being monitored, or other person whose health condition is to be monitored. During the training phase, the human subject is one receiving a vaccination for the communicable disease of interest (or for another communicable disease expected to have similar onset symptoms). In this case, the monitoring system 1 is worn for a suitable time interval bracketing the time of vaccination, e.g., a day or two before the vaccination through to two or three days after the time of vaccination (or longer, or shorter, depending on the expected time interval for physiological reaction to the vaccination). The physiological data collected before the vaccination advantageously serves as baseline data during which there should be no reaction to the (not yet administered) vaccination, while the physiological data collected after the vaccination reflects the subject's physiological (and potentially also psychosomatic) reaction to the vaccination. In the case of a placebo, the physiological data collected after the vaccination reflects only any psychosomatic reaction to the vaccination.

As shown in FIG. 1, the system 1 includes a wearable monitoring device 10 that is wearable by, or otherwise attached to, the human subject S. The wearable monitoring device 10 (diagrammatically shown in FIG. 1 as a rectangle attached to a portion of the human subject S) can include any suitable monitoring device, such as a Mobile Cardiac Outpatient Telemetry (MCOT)^{®} device (available from Philips ECG Solutions), an Oura Ring wearable monitor (available from Oura Health Oy, Oulu, Finland), a Zephyr^{™} wearable monitor (available from SenLab, Ljubljana, Slovenia), or a medical wearable device, a torso-worn vital signs health patch, a wrist-worn watch, a chest strap, a smart garment, medical ear buds/over the ear, a forehead or nose sensor, a smart ring, or so forth.

The illustrative wearable monitoring device 10 can include one or more sensors 12 configured to measure physiological sensor data 13 of the human subject S wearing the monitoring device 10. The measured physiological sensor data 13 of the human subject S is used to detect a health-related physiological parameter of the patient P. For example, the monitoring device 10 can include a heart rate (HR) sensor 12 configured (e.g., comprising skin-contacting electrodes with silver/silver chloride coatings, for example) to measure cardiac data 13 of the human subject S. In addition, the HR sensor 12 can be replaced with (or supplemented by) any other suitable sensor to measure a corresponding vital sign of the patient (e.g., temperature SpOz, electroencephalogram (EEG), and so forth). In another example, the monitoring device 10 can include a respiration rate (RR) sensor 12 configured to measure respiratory data 13 of the human subject S. While HR, ECG, or RR data is described herein as an illustrate example, the monitored vital sign data could additionally or alternatively be respiratory rate (RR) data, SpOz data, activity data (e.g., acquired by a wearable accelerometer), or so forth, or various combinations thereof.

The illustrative wearable monitoring device 10 also includes a wireless transmitter or transceiver 14 (referred to hereinafter as a transceiver 14) integrated with or in wireless communication with the monitoring device 10 to transmit the physiological sensor data 13 to a clinical health information system comprising a server computer 20, for example transmitted over the Internet via a 3G/4G/5G wireless cellular network, Wi-Fi, various combinations thereof, and/or another wireless communication protocol. In a typical arrangement, the transceiver 14 is a low-power wireless transceiver (e.g., Bluetooth^{™}, Zigbee^{™}, or the like) that connects with low power to the server computer 20, thus placing a low power draw on the on-board battery of the wireless monitoring device 10. The server computer 20 can also include an electronic module or processor 21 configured to execute instructions for determining whether the human subject Shas received one or more vaccinations. In the case of the training phase, it is contemplated to omit the transceiver 14 and to instead store the physiological data on the monitoring device 10 and offload it after the time interval after the vaccination has ended. For example, the training subject may receive and wear the monitoring device 10 from a day before the vaccination (or placebo) until three days after the vaccination (or placebo) after which time the training subject returns the physical monitoring device 10 to the persons conducting the training who then offload the training data using a USB port or other wired connection.

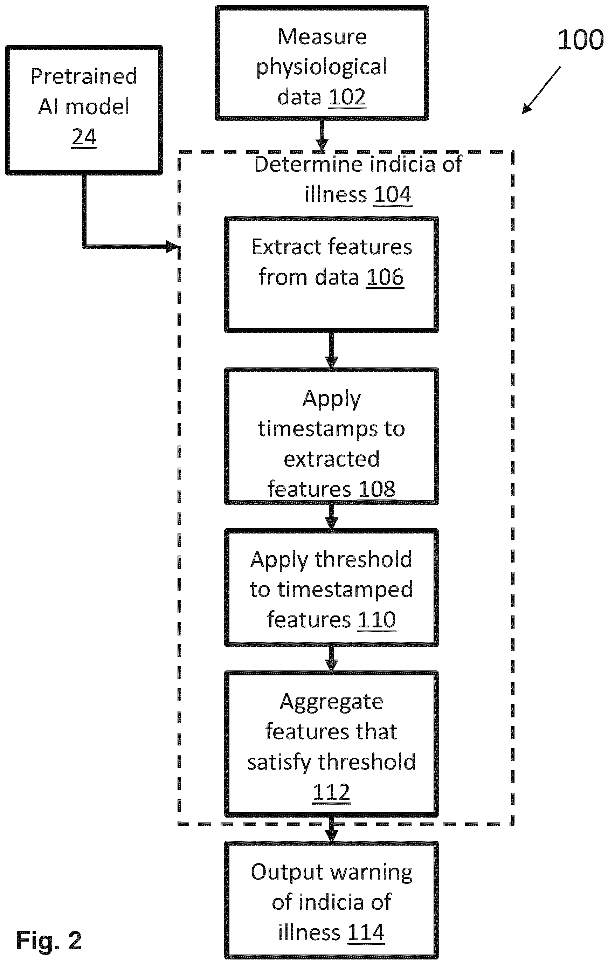

With reference now to FIG. 2, an illustrative embodiment of the vaccination status monitoring method 100 is shown by way of a flowchart. The vaccination status monitoring method 100 illustrates the inference phase, that is, the use of the trained AI model to detect early onset of the communicable disease of interest. To begin the method 100, the monitoring device 10 is attached to the human subject S. At an operation 102, physiological sensor data 13 is measured by the at least one sensor 12 of the monitoring device 10, and is transmitted via the transceiver 14 for receiving by the server computers. The sensor(s) 12 of the monitoring device 10 is configured to measure the physiological sensor data 13 of the human subject S, in some embodiments including at least two of heart rate data, respiration data, and temperature data.

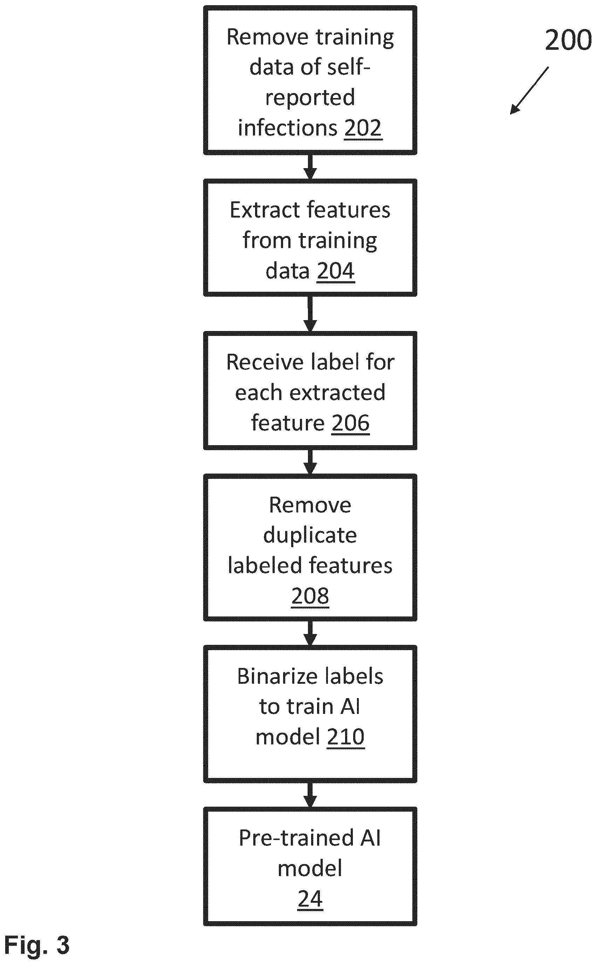

At an operation 104, the server computer 20 is configured to determine whether the human subject S has indicia of onset an illness by applying a pre-trained artificial intelligence (AI) model 24 to the received physiological sensor data 13. The pre-trained AI model 24 is pre-trained using a method 200 (see FIG. 3 and related discussion for the training) to determine whether the human subject S has received a vaccination, for example, against the illness. As will be discussed further with reference to FIG. 3, the training performed to generate the pre-trained AI model 24 utilizes training data of training subjects who receive a vaccination for the communicable disease of interest (or a related disease with similar onset symptoms). In some embodiments, the pre-trained AI model 24 comprises a pre-trained decision tree model. In a particular embodiment, the pre-trained decision tree model 24 comprises an XGBoost model, which advantageously allows a user to see which features from the received physiological sensor data 13 contribute most to an output of the determination whether the human subject S has indicia of onset an illness. In some embodiments, the illness is pneumococcal infection and the pre-trained AI model 24 is pre-trained to determine whether the associated human subject has received a pneumococcal infection vaccination. In other embodiments, the illness is a coronavirus (COVID) infection and the pre-trained AI model 24 is pre-trained to determine whether the associated human subject has received a COVID vaccination.

Referring now to FIG. 3, and with continuing reference to FIG. 2, an illustrative embodiment of an AI model training method 200 is shown by way of a flowchart. The method 200 can be implemented by the server computer 20 to train an AI model on training physiological sensor data acquired of a cohort of training subjects over a training period to produce the pre-trained AI model 24. In some embodiments, the cohort of training subjects includes (i) training subjects who received a vaccination injection against the illness during the training period, and (ii) training subjects who did not receive a vaccination injection against the illness during the training period and who did receive a placebo injection during the training period wherein the placebo injection does not vaccinate against the illness. This training approach is based on the expectation that the physiological reaction of the training subject to the vaccine is similar to physiological symptoms of onset of the actual disease. This expectation is reasonable since the vaccination typically includes an active agent such as the microorganism that causes the communicable disease of interest, usually in a weakened or killed state. Hence, both the vaccination and an early onset of the communicable disease are expected to trigger a similar immune response from the subject.

The server computer 20 is configured to preprocess the training physiological sensor data before training the AI model on the training physiological sensor data in the method 200. At an operation 202, training physiological sensor data acquired of any training subject of the cohort of training subjects having self-reported an infection is removed from the training physiological sensor data. This is done to remove any suspicious data, for example due to a training subject who actually contracts the communicable disease of interest before the training subject's scheduled vaccination. This results in generating a cohort of the training physiological sensor data to train the AI model to generate the pre-trained AI model 24.

At an operation 204, features from the training physiological sensor data are extracted. The features can include, for example, statistics of the training physiological sensor data, a trend of statistics of the training physiological sensor data, trends of statistics of the training physiological sensor data over different window sizes, and a sleep duration extracted from the training physiological sensor data.

At an operation 206, a label for each extracted feature of the training physiological data is received by the server computer 20, and at an operation 208, duplicate labeled features are removed from a set of the extracted features. At an operation 210, the label received for each extracted feature is binarized (e.g., on a 0, 1, or 2 scale). Cross-validation processes with various window sizes utilizing personalized features are used to identify vaccination group labels. These binarized values for each extracted feature are input to the AI model to generate the pre-trained AI model 24.

Referring back to FIG. 2, the determination operation 104 includes a series of sub-operations to determine whether the human subject S has indicia of onset of an illness. At an operation 106, physiological sensor data features are extracted from the received physiological sensor data 13. Physiological sensor data acquired of any human subject S having self-reported an infection is removed from the received physiological sensor data 13. The physiological sensor data features in some embodiments comprise two or more of: statistics of the received physiological sensor data 13, a trend of statistics of the received physiological sensor data 13, trends of statistics of the received physiological sensor data 13 over different window sizes, and a sleep duration extracted from the physiological sensor data 13. Several different techniques can be used to derive the features including pooling over two-week periods, trend statistics to capture behavior over several days, baseline corrections that compare baseline distributions in the days prior to vaccination with the corresponding distributions after the vaccine has been administered.

The extracted physiological sensor data features satisfying a predetermined threshold are then aggregated. To do so, at an operation 108, timestamps are applied to the extracted physiological sensor data features. The timestamp manipulations to quantize timestamp resolution of the received physiological sensor data 13 from seconds to days. At an operation 110, the predetermined threshold is applied to the timestamped physiological sensor data features. The predetermined threshold is applied on the coverage of each extracted feature to take features with enough coverage (less a missing rate of coverage). If a certain feature has too large of a missing rate, then an algorithm with a built-in imputation mechanism could end up imputing a large percentage of values, which could add additional bias to the output results. The cut-off of the feature coverage threshold is one of the hyper-parameters. The threshold can be, for example, 0.6 as the feature coverage threshold. At an operation 112, the timestamped physiological sensor data features satisfying the predetermined threshold are aggregated, i.e., by taking a mean value of the extracted features to obtain on value per feature each day.

At an operation 114, a warning 26 can be output in response to a determination that the human subject S has indicia of onset of the illness. Referring to FIG. 1, an electronic processing device 18 (e.g., a smart phone, a smart tablet, or a computer) can be in electronic communication with the server computer 20. The electronic processing device 18 includes a display device 22 (e.g., a touchscreen) for displaying a representation of the warning 26. The warning 26 can look at the true positive rate of 10-fold cross-validation results, for example.

The disclosed approach for training an AI model to provide early detection of onset of a communicable disease was tested using the surrogate of inactive pneumococcal and typhoid infection vaccination, along with placebo. The resulting trained AI model is expected to be useful for detecting early onset of pneumococcal infection, and possibly other diseases with similar early onset symptoms such as COVID. In this experimental AI model training, a less strong physiological response from vaccination was expected compared to COVID infection, however, in order to eliminate the confounding physiological effects from infections rather than a vaccine or placebo shot, subjects with any self-reported infection were eliminated (corresponding to FIG. 3, operation 202).

The features in the AI model were for the most part the same as in the one for the Rapid Analysis of Threat Exposure (RATE) algorithm [Bryan, Conroy, et al, Real-time infection prediction with wearable physiological monitoring and AI to aid military workforce readiness during COVID-19 https://www.nature.com/articles/s41598-022-07764-6]. The features are derived from basic vitals from wearables such as temperature, heart rate, and respiration rate measurements. Then secondary features are extracted by looking at statistics per sleep period, such as Mean Heart Rate. Timestamp manipulations were applied to quantize timestamp resolution from seconds to days. A feature threshold is applied on the coverage of each feature to take features with enough coverage (less missing rate). If a certain feature has too large a missing rate, then an algorithm with a built-in imputation mechanism ended up imputing a large percentage of values, which could add additional bias to the output results. The cut-off of the feature coverage threshold is one of the hyper-parameters and in this experiment, 0.6 was used as the feature coverage threshold. Finally, feature aggregation was applied by taking the mean on those daily features to obtain one value per feature each day. Preprocessing steps of getting vaccine group label from survey included taking the label information of each subject from Data Access Object (DAO) and first taking completed studies, then taking unique labels and subjects to remove duplicates in the label data frame, then binarizing labels from group 0, 1, 2 to zero-one hot vectors, as target values for the machine learning of the AI model. Vital signs of temperature, heart rate, and heart rate variabilities were primarily taken from Oura ring, and respiratory rates from Zephyr. Those signals were passed through feature generation to obtain a set of Derived features list. Those derived features are statistics of vital signs, and an example would be mean Heart Rate. As further examples, suitable statistical features can include, but are not limited to count, min, mean, max, median, standard deviation, Inter-quartile range, Quartile coefficient of dispersion, percentiles; trend statistical features; monotonic trends with different window sizes, the number of paired nights contribute to trend features; sleep duration; and so forth. Labels were utilized from vaccination groups (2 groups are Pneumococcal and typhoid inactive vaccines and the other group is a placebo) and build an XGBoost model that mapped features to vaccination groups. Cross-validation was used with various window sizes utilizing personalized features to identify vaccination group labels.

In some embodiments, the wearable monitoring device 10 can include an electronic processor 16 integrated with the wearable monitoring device 10. The electronic processor 16 can optionally perform some of the operations in the method 100 (e.g., pre-processing the physiological sensor data 13 before transmission by the transceiver 14 to the server computer 20).

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A system (1) comprising:
an electronic processor (16, 20) integrated with or in wireless communication with an associated monitoring device (10), the electronic processor programmed to:
receive physiological sensor data (13) of an associated human subject measured using the associated monitoring device (10);
determine whether the associated human subject has indicia of onset an illness by applying a pre-trained artificial intelligence (AI) model (24) to the received physiological sensor data; and
output a warning (26) in response to a determination that the associated human subject has indicia of onset of the illness;
wherein the pre-trained AI model is pre-trained to determine whether the human subject has received a vaccination.

2. The system (1) of claim 1, wherein the electronic processor (16, 20) is further programmed to:
extract physiological sensor data features from the received physiological sensor data (13);
aggregate the extracted physiological sensor data features satisfying a predetermined threshold; and
apply the pre-trained AI model (24) to the aggregated physiological sensor data features to.

3. The system (1) of claim 2, wherein the electronic processor (16, 20) is further programmed to:
apply timestamps to the extracted physiological sensor data features;
apply the predetermined threshold to the timestamped physiological sensor data features; and
aggregate the timestamped physiological sensor data features satisfying a predetermined threshold.

4. The system (1) of either one of claims 2 and 3, wherein the physiological sensor data features comprise two or more of: statistics of the received physiological sensor data (13), a trend of statistics of the received physiological sensor data (13); trends of statistics of the received physiological sensor data over different window sizes, and a sleep duration extracted from the physiological sensor data.

5. The system (1) of any one of claims 1-4, wherein the illness is pneumococcal infection, and the pre-trained AI model (24) is pre-trained to determine whether the associated human subject has received a pneumococcal infection vaccination.

6. The system (1) of any one of claims 1-4, wherein the illness is a coronavirus (COVID) infection, and the pre-trained AI model (24) is pre-trained to determine whether the associated human subject has received a COVID vaccination.

7. The system (1) of any one of claims 1-6, wherein the pre-trained AI model (24) is pre-trained to determine whether the associated human subject has received a vaccination against the illness.

8. The system (1) of any one of claims 1-7, wherein the associated monitoring device (10) is configured to measure physiological sensor data (13) of the associated human subject including at least two of heart rate data, respiration data, and temperature data.

9. The system (1) of any one of claims 1-8, wherein the pre-trained AI model (24) comprises a pre-trained decision tree model.

10. The system (1) of any one of claims 1-9, further comprising:
a server computer (20) programmed to train an AI model on training physiological sensor data acquired of a cohort of training subjects over a training period to produce the pre-trained AI model (24).

11. The system (1) of claim 10, wherein the cohort of training subjects includes:
training subjects who received a vaccination injection against the illness during the training period, and
training subjects who did not receive a vaccination injection against the illness during the training period and who did receive a placebo injection during the training period wherein the placebo injection does not vaccinate against the illness.

12. The system (1) of either one of claims 10 and 11, wherein the server computer (20) is programmed to preprocess the training physiological sensor data before training the AI model on the training physiological sensor data, the preprocessing including:
extracting features from the training physiological sensor data;
receiving a label for each extracted feature;
removing duplicate labeled features from a set of the extracted features; and
binarizing the label received for each extracted feature.

13. The system (1) of any one of claims 10-12, wherein the server computer (20) is programmed to preprocess the training physiological sensor data before training the AI model on the training physiological sensor data, the preprocessing including:
removing training physiological sensor data acquired of any training subject of the cohort of training subjects having self-reported an infection.

14. The system (1) of any one of claims 1-13, further comprising:
a monitoring device (10) including at least one sensor (12), the monitoring device configured to be worn by the associated human subject and the at least one sensor configured to measure physiological sensor data (13) of the associated human subject wearing the monitoring device.

15. A vaccination status monitoring method (100), comprising:
receiving physiological sensor data (13) of an associated human subject measured using an associated monitoring device (10);
determining whether the associated human subject has indicia of onset an illness by applying a pre-trained artificial intelligence (AI) model (24) to the received physiological sensor data; and
outputting a warning (26) in response to a determination that the associated human subject has indicia of onset of the illness;
wherein the pre-trained AI model is pre-trained to determine whether the human subject has received a vaccination.
